# EUROPÄISCHE PATENTSCHRIFT

(11) **EP 2 168 564 B1**
(45) Veröffentlichungstag und Bekanntmachung des Hinweises auf die Patenterteilung: **21.11.2018**
(21) Anmeldenummer: 09168540.4
(22) Anmeldetag: 25.08.2009
(51) Int. Cl.: A61K 8/06, A61K 8/895, A61K 8/90, A61Q 19/00, A61Q 19/04, A61Q 19/08, A61Q 17/04, A61K 8/894, A61K 8/02

(54) **Emulgatorsysteme für kosmetische und pharmazeutische Öl-in-Wasser-Emulsionen**
Emulsifier systems for cosmetic and pharmaceutical oil-in-water emulsions
Système d'émulsifiant pour émulsions cosmétiques et pharmaceutiques huile dans l'eau

(30) Priorität: 26.09.2008 DE 102008042381
(43) Veröffentlichungstag der Anmeldung: 31.03.2010
(73) Patentinhaber: Evonik Degussa GmbH, 45128 Essen (DE)
(72) Erfinder: Meyer, Jürgen, 45134, Essen (DE); Hartung, Christian, 45133, Essen (DE); Czech, Karin, 45309, Essen (DE); Ferenz, Michael, 45147, Essen (DE); Lohse, Andrea, 46236, Bottrop (DE); Herrwerth, Sascha, 45134, Essen (DE); Unger, Frank, 47167, Duisburg (DE); Grüning, Burghard, 45134, Essen (DE)

(56) Entgegenhaltungen:
- EP-A1- 2 022 812
- EP-A2- 1 069 129
- EP-A2- 1 125 574
- WO-A1-2004/014324
- WO-A1-2004/039338
- WO-A1-2009/138306
- WO-A2-2010/118926
- nn: "Abil Care XL 80", Slovakia , 1. Februar 2008 (2008-02-01), Seiten 1-9, XP002722774, Gefunden im Internet: URL:http://www.finecon.sk/admin/pdf/DS_ABI L_Care_XL_80.pdf [gefunden am 2014-04-02]
- Hans Dr. Kreul: "New Evonik Products at PCHI in Guangzhou", , 27. Januar 2008 (2008-01-27), Seiten 1-5, XP055000310, Gefunden im Internet: URL:http://corporate.evonik.com/_layouts/W ebsites/Internet/NewsAttachmentHandlerSec. ashx?fileid=6581&newsid=13152&NewsSecToken =XlE1b%2bRv0NCol6AjjDybyHUV9hBxwHJvXAD%2fV ALLBVL3iGjNuflZyq1MOhvo1hXZ&AllowNews=True [gefunden am 2011-06-08]
- DIETZ T: "Ein neuartiger Silikon-O/W-Emulgator mit Hautglättegefühl", SOFW-JOURNAL SEIFEN, OELE, FETTE, WACHSE, VERLAG FUR CHEMISCHE INDUSTRIE, AUGSBURG, DE, Bd. 128, Nr. 8, 1. Januar 2002 (2002-01-01), Seiten 22-32, XP002231678, ISSN: 0942-7694

## Beschreibung

### Gebiet der Erfindung

Gegenstand der Erfindung sind Emulgator-Systeme für kosmetische und pharmazeutische Öl-in-Wasser-Emulsionen enthaltend hochmolekulare organomodifizierte Polysiloxane mit einer Polydispersität größer 1,6, kosmetische und pharmazeutische Öl-in-Wasser-Emulsionen und kosmetische und pharmazeutische Kompositionen.

### Stand der Technik

Organomodifizierte Siloxane werden in den verschiedensten Applikationen eingesetzt. Ihre Eigenschaften lassen sich unter anderem durch die Art der Modifikation, sowie durch die Modifikationsdichte gezielt einstellen.

So können zum Beispiel mit Allylpolyethern organophile oder nichtionische hydrophile Gruppen an ein Siloxangerüst gebunden werden. Derartige Verbindungen finden ihren Einsatz zum Beispiel als Polyurethan-Schaum-Stabilisatoren, als Entschäumer in Treibstoffen oder als Additive in Farben und Lacken.

So beschreibt z.B. DE 102005001041 funktionalisierte Polyorganosiloxane und deren Einsatz als Kraftstoffentschäumer. Die Allylpolyether in den hier dargestellten Siloxanen können gegebenenfalls durch Abänderung der Synthese durch Kohlenwasserstoffreste ersetzt werden.

Generell können Siloxane durch Umsetzung mit z.B. α-Olefinen mit hydrophoben Gruppen verknüpft werden. Die so erhaltenen Siliconwachse dienen zum Beispiel als Additiv in Personal-Care-Applikationen.

Es zeigt sich in vielen Anwendungsgebieten, dass die Wirkung des Siloxans entscheidend von der Verträglichkeit mit der entsprechenden Formulierung abhängt.

Als kosmetischer Emulgator geeignet sind zum Beispiel Siloxane, die neben aliphatischen Gruppen auf Basis von α-Olefinen Polyether tragen. Als typisches Beispiel ist hier das Verkaufsprodukt ABIL EM 90 der Evonik Goldschmidt GmbH (Deutschland) zu nennen, das sich insbesondere durch eine hervorragende Stabilisierung von Wasser-in-Öl-(W/O)-Emulsionen auszeichnet (US 4698178).

Siloxanbasierte Emulgatoren für Öl-in-Wasser-(O/W)-Emulsionen müssen einen hydrophileren Charakter aufweisen, weshalb es sich bei diesen Produkten in der Regel um reine Polyethersiloxane handelt.

EP 1125574 beschreibt die Verwendung relativ hydrophober Polyethersiloxane als O/W-Emulgatoren, bei denen sich die Polyethergruppen α-ω-ständig am Siloxanrücken befinden. Diese Strukturen zeichnen sich insbesondere durch ein samtig seidiges Hautgefühl aus, das sie in kosmetische Emulsionen einzubringen vermögen.

Nachteilig an der Verwendung dieser Strukturen sind die oft nicht ausreichende Emulsionsstabilisierung sowie die aufwendige Herstellung aufgrund der anspruchsvollen Topologie.

Aufgabe der Erfindung ist es, Emulgator-Systeme zur Verfügung zu stellen, die in der Lage sind, ein samtig-seidiges Hautgefühl zu erzielen und dabei eine hohe Emulsionsstabilisierung aufweisen.

### Beschreibung der Erfindung

Überraschenderweise wurde gefunden, dass hochmolekulare organomodifizierte Siloxane bzw. Mischungen verschiedener hochmolekularer organomodifizierter Siloxane als emulgieraktive Komponenten wirken und dabei in Verbindung mit einem guten Hautgefühl besonders stabile Öl-in-Wasser-Emulsionen liefern, wenn sie eine ungewöhnlich breite Molekulargewichtsverteilung aufweisen.

Überraschenderweise ist bei hochmolekularen Polyethersiloxanen mit breiter Molekulargewichtsverteilung ein relativ geringer Substitutionsgrad mit hydrophilen Polyethergruppen bereits in der Lage, hervorragende Emulgierperformance zu erzielen, während gleichzeitig ein samtig-seidiges Hautgefühl erreicht wird.

Die erfindungsgemäßen organomodifizierten Polysiloxane oder Polyethersiloxanmischungen werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können.

Werden im Rahmen der vorliegenden Erfindung Verbindungen, wie z.B. organomodifizierte Polysiloxane, beschrieben, die verschiedene Einheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als Mittelwert, gemittelt über alle entsprechenden Verbindungen, zu verstehen. Alle angegebenen Prozent (%) sind, wenn nicht anders angegeben, Massenprozent.

Gegenstand der vorliegenden Erfindung sind daher Emulgator-Systeme für kosmetische und pharmazeutische Öl-in-Wasser-Emulsionen enthaltend hochmolekulare organomodifizierte Polysiloxane mit einer Polydispersität D größer 1,6, wie in

### Anspruch 1 beschrieben.

Bei der Polydispersität D handelt es sich um den Quotienten aus dem Gewichtsmittel Mw und dem Zahlenmittel Mn der Molgewichtsverteilung der Polysiloxane. Die Polydispersität ist ein anerkannter Maßstab für die Breite einer Molmassenverteilung. Polysiloxane weisen typischerweise eine Polydispersität von D kleiner 1,6 auf.

Eine übliche Methode zur Ermittlung der Molgewichtsverteilung ist die Gelpermeationschromatographie (GPC) .

Die in der vorliegenden Anmeldung verwendete GPC-Methode ist analog zur Norm DIN 55072-1 / ISO 13885-1. Die GPC-Daten wurden an einem Hewlett Packard HP 1100 Gerät mit HP RI-Detektor und folgenden Parametern erhalten:
- Säule:
   SDV1000/10000Å,
   Länge: 65,00 cm,
   Innendurchmesser: 0,80 cm,
   Temperatur: 30 °C
- Mobile Phase: THF
- Fließrate: 1,00 ml/min
- Probenkonzentration: 10,00 g/l
- Kalibrierung: gegen PS [162-2057000 g/mol].

Für die Auswertung der Chromatogramme wurde die Auswertesoftware WinGPC Unity von Polymer Standards Service, Mainz, Deutschland, eingesetzt.

Für die vorliegenden Daten wurde nur das Produktsignal im GPC-Chromatogramm betrachtet. Handelt es sich bei den Polysiloxanen um Polyethersiloxane, die mittels Hydrosilylierung hergestellt wurden, enthalten diese üblicherweise als Nebenbestandteil einen gewissen Anteil freien Polyether. Im GPC erzeugen diese Signale, welche gegebenenfalls den Produktpeak überlagern. Dementsprechend wurde eine standardmäßige Multipeak-Auswertung analog zu HPLC-Auswertungen angewendet und nur das Produktsignal betrachtet. Sofern andere Signale bei niedrigeren Molmassen dieses Produktsignal überlagert haben, wurde mit Hilfe der Auswertesoftware das Minimum zwischen den Signalen bestimmt, eine Fällung auf die Basislinie durchgeführt und nur ab dem Molekulargewicht des Minimums das Chromatogramm zu größeren Molmassen ausgewertet.

Die Polydispersität D der in dem erfindungsgemäßen Emulgator-System eingesetzten Polysiloxane ist größer 1,6, bevorzugt größer 1,7 und besonders bevorzugt größer 1,8 (bestimmt für das Produktsignal nach dem oben aufgeführten GPC-Verfahren) .

Unter "hochmolekulare Polysiloxane" im Sinne der vorliegenden Erfindung werden Polysiloxane verstanden, die ein Gewichtsmittel Mw von mindestens 5000 g/mol, bevorzugt von mindestens 10000 g/mol und besonders bevorzugt von 15000 g/mol aufweisen (bestimmt für das Produktsignal nach dem oben aufgeführten GPC-Verfahren).

Bei den erfindungsgemäßen Emulgator-Systemen kann es sich um ein einzelnes organomodifiziertes Polysiloxan mit einer breiten Molgewichtsverteilung als auch um Mischungen von organomodifizierten Polysiloxanen unterschiedlicher Molekulargewichtsverteilungen handeln.

Besonders vorteilhaft sind Polysiloxane mit einer breit verteilten Molekulargewichtsverteilung, die zudem signifikante hochmolekulare Anteile enthalten.

Somit werden in dem erfindungsgemäßen Emulgator-System Polysiloxane eingesetzt, die einen Anteil an Polysiloxanen mit einem Molekulargewicht von ≥ 1*10⁴ g/mol von über 75%, bevorzugt von über 80% und besonders bevorzugt von über 85% bezogen auf die Gesamtmenge des Polysiloxans aufweisen, und
enthaltend Polysiloxane mit einem Anteil an Polysiloxanen mit einem Molekulargewicht von ≥ 1*10⁵ g/mol von über 2%, bevorzugt von über 2,5% und besonders bevorzugt von über 5%, bezogen auf die Gesamtmenge des Polysiloxans.

Ein zu hohes Molekulargewicht des in dem erfindungsgemäßen Emulgator-System eingesetzten Polysiloxans kann nachteilige Auswirkungen auf die Emulgierleistung ausüben, somit werden in dem erfindungsgemäßen Emulgator-System bevorzugt Polysiloxane eingesetzt, die einen Anteil an Polysiloxanen mit einem Molekulargewicht von ≥ 1*10⁷ g/mol von weniger 5%, bevorzugt von weniger 2% und besonders bevorzugt von weniger 1% bezogen auf die Gesamtmenge des Polysiloxans aufweisen.

Der Anteil der Polysiloxane eines bestimmten Molekulargewichts wird bestimmt über die jeweiligen Flächenanteile des jeweiligen Polysiloxans eines bestimmten Molekulargewichts nach dem oben aufgeführten GPC-Verfahren. Der Flächenanteil wird bezogen auf die Fläche aller Polysiloxane (= Gesamtmenge des Polysiloxans); somit sind die hier angegebenen % Flächen-%.

Bevorzugte Emulgator-Systeme enthalten dabei mindestens ein Polyethersiloxan.

Bevorzugte Polyethersiloxan enthaltende Emulgator-Systeme enthalten Polyethersiloxane der allgemeinen Formel I

M_{2+c+2d} Dₐ D'_{b} T_{c} Q_{d} Formel I,

wobei
M = (R¹R²₂ SiO_{1/2})
D = (R²₂ Si O_{2/2})
D' = (R²R³ Si O_{2/2})
T = (R² Si O_{3/2})
Q = (Si O_{4/2}) a = 30 - 800, bevorzugt 40 bis 500, insbesondere 50 bis 400,
b = 1 bis 15, bevorzugt 3 bis 10, insbesondere 4 bis 8,
c = 0 bis 2, bevorzugt 0 bis 1, insbesondere 0,
d = 0 bis 2, bevorzugt 0 bis 1, insbesondere 0,
R¹ = R² oder R³,
R² = unabhängig voneinander gleiche oder verschiedene lineare oder verzweigte, gegebenenfalls aromatische Kohlenwasserstoffreste mit 1 bis 16 Kohlenstoffatomen, die gegebenenfalls OH- oder Esterfunktionen tragen, bevorzugt Methyl oder Phenyl, insbesondere Methyl,
R³ = unabhängig voneinander gleiche oder verschiedene Polyetherreste der allgemeinen Formel II:

   -CH₂-CH₂-(CH₂)ₙO(EO)ₓ(PO)_{y}(XO)_{z} R⁴ Formel II,

   mit
   EO = (C₂H₄O)
   PO = (C₃H₆O)
   XO = (C₂H₃R⁵O)
   n = 1 - 9, insbesondere 1
   x = 2 - 50, insbesondere 10-30
   y = 0 - 50, insbesondere 2-15
   z = 0 - 10, insbesondere 0
   R⁴ = unabhängig voneinander gleiche oder verschiedene Reste ausgewählt aus der Gruppe umfassend: H, Alkylreste mit 1 bis 16 C-Atomen, oder Carboxylatreste, bevorzugt H oder Methyl, und
   R⁵ = unabhängig voneinander gleiche oder verschiedene Reste ausgewählt aus der Gruppe umfassend:
      Alkylreste mit 2 bis 16 C-Atomen, die gegebenenfalls durch Etherfunktionen unterbrochen sind, Alkarylreste mit 7 - 18 C-Atomen, Arylreste mit 6 bis 16 C-Atomen, bevorzugt Ethyl oder Phenyl, ist.

Die Verbindungen liegen in Form eines Gemisches vor mit einer im Wesentlichen durch statistische Gesetze geregelten Verteilung. Die Werte für die Indizes x, y und z stellen deshalb Mittelwerte dar. Die mit den Indizes x, y und z gekennzeichneten Einheiten können in den Verbindungen der Formel II statistisch verteilt, blockweise oder in jeder anderen beliebigen Reihenfolge angeordnet vorliegen.

In den erfindungsgemäßen Emulgator-Systemen können Polyethersiloxane mit relativ hydrophilen Polyethern eingesetzt werden, ohne das samtig-seidige Hautgefühl der Emulgator-Systeme in kosmetischen und dermatologischen Formulierungen negativ zu beeinflussen. Gleichzeitig zeichnen sich solche Moleküle aber durch eine besonders gute Emulsionsstabilisierung aus.

Daher enthalten erfindungsgemäße Emulgator-Systeme bevorzugt Polyethersiloxane mit einem hohem EO-Anteil für deren Polyetherkomponente gemäß Formel II gilt:
x/(y+z) > 1, bevorzugt > 2 und besonders bevorzugt > 3.

Es kann vorteilhaft für die Emulgiereigenschaften und das Hautgefühl sein, wenn in den eingesetzten Polyethersiloxanen der Anteil unmodifizierter D-Einheiten deutlich größer als der Anteil modifizierter D'-Einheiten ist.

Somit enthalten erfindungsgemäße Emulgator-Systeme bevorzugt Polyethersiloxane, die sich dadurch auszeichnen, dass das Verhältnis a/b (aus Formel I) > 5, bevorzugt > 8 und besonders bevorzugt > 10 ist.

Es kann für das Hautgefühl besonders vorteilhaft sein, wenn Polyethersiloxane mit einem relativ niedrigen Polyetheranteil eingesetzt werden.

Erfindungsgemäße Emulgator-Systeme enthalten daher bevorzugt Polyethersiloxane der allgemeinen Formel I, die im Schnitt mindestes drei Polyetherreste enthalten und bei denen die maximale Anzahl der ans Molekül gebundenen Polyetherreste R³ < a/5 ist.

Polyethersiloxane für den Einsatz in den erfindungsgemäßen Emulgator-Systemen sind auf verschiedene Art und Weise zugänglich. Im Allgemeinen werden sie durch die Hydrosilylierung von allylfunktionellen Polyethern an SiHfunktionelle Siloxane gewonnen. Derartige Verbindungen sind zum Beispiel in EP 1754740 beschrieben.

Die zugrunde liegenden SiH-funktionellen Siloxane werden im Allgemeinen durch die Equilibrierung von verschiedenen Siloxangrundkörpern gewonnen. Verfahren zur Equilibrierung sind zum Beispiel in den Patenschriften EP 1439200 und DE 102005001039 beschrieben, auf welche ausdrücklich verwiesen wird. Im industriellen Maßstab werden zur Darstellung von SiH-Gruppen tragenden Organopolysiloxanen vorzugsweise leicht zugängliche Siloxanverbindungen, wie zum Beispiel Decamethylcyclopentasiloxan, Poly(methylwasserstoff)siloxane, 1,1,3,3-Tetramethyldisiloxan oder Hexamethyldisiloxan, in Gegenwart eines geeigneten Katalysators zur Umsetzung gebracht. Geeignete Katalysatoren sind starke Säuren, wie Trifluormethansulfonsäure. Dabei bilden sich die entsprechenden Equilibrate. Die SiH-Funktionalitäten können je nach eingesetztem Katalysator statistisch über die Siloxanhauptkette verteilt vorliegen aber auch blockweise auftreten. Auch der Funktionalitätsgrad der einzelnen Polymermoleküle unterliegt einer Verteilung. Die Indizes a, b, c und d der im Rahmen dieser Erfindung eingesetzten Siloxane stellen deshalb Mittelwerte dar. Die mit den Indizes a, b, c und d gekennzeichneten Einheiten können in den Verbindungen der Formel I statistisch verteilt, blockweise oder in jeder anderen beliebigen Reihenfolge angeordnet vorliegen.

Als Katalysator für die Hydrosilylierungsreaktion werden insbesondere Platin und seine Verbindungen eingesetzt. Dabei wird das Platin entweder in metallischer Form, als auf einen Träger fixiertem Metall oder in Form eines gegebenenfalls löslichen Platinkomplexes eingesetzt. Bis heute wird ein Großteil der industriell durchgeführten Hydrosilylierungsreaktionen mit dem aus den US 3715334 und US 3775452 bekannten sog. Karstedt-Katalysator durchgeführt.

Die Darstellung der erfindungsgemäßen Polyethersiloxane kann lösemittelfrei erfolgen. Der Einsatz eines Lösemittels ist unter Umständen jedoch vorteilhaft bzw. notwendig. So sind die Polyether in der Regel unverträglich mit dem Siloxan und durch den Einsatz eines Lösemittels lässt sich vermeiden, dass die Initiierung der Reaktion verzögert eintritt.

Die technische Reaktionsführung zur Herstellung der Polyethersiloxane kann die Eigenschaften des Produktes beeinflussen, insbesondere wenn mehrere verschiedene Polyetherreste angelagert werden. Erfindungsgemäße Produkte lassen sich unter anderen sowohl in diskontinuierlich, halbkontinuierlich als auch kontinuierlich betriebenen Kesseln herstellen.

Geeignete Verfahren zur Hydrosilylierung werden zum Beispiel in dem Buch "Chemie und Technologie der Silicone", Verlag Chemie, 1960, Seite 43, und in DE 2646726, US 3775452 und EP 1520870 beschrieben, auf welche ausdrücklich verwiesen wird.

Für die Herstellung der erfindungsgemäßen Polyethersiloxane lassen sich verschiedenartige Polyether einsetzen. Diese werden in der Regel durch die Anlagerung von Alkoxiden an ein- oder mehrfach-funktionelle Alkohole oder ein Amin hergestellt. Aufgrund ihrer guten kommerziellen Verfügbarkeit eignen sich für den Aufbau von Polyethern insbesondere die Alkoxide: Ethylenoxid, Propylenoxid, Butylenoxid oder Styroloxid.

Werden verschiedene Monomere zur Herstellung der Polyether verwendet, zum Beispiel um gezielt die Hydrophilie des Produktes einzustellen, so lässt sich durch die Reihenfolge der Zudosierung und durch die Einstellung verschiedener Reaktionsparameter die Verteilung der Monomereinheiten entlang der Polymerhauptkette steuern, so dass zum Beispiel unterschiedliche Monomereinheiten blockweise auftreten können oder graduell bzw. statistisch verteilt vorliegen. Als Polyether können auch solche Polyether eingesetzt werden, die durch das Verfahren der Pfropfpolymerisation weiter modifiziert worden sind. Dazu werden die Polyether in Gegenwart radikalischer Aktivatoren mit Doppelbindungen tragenden Monomeren umgesetzt. Durch die Einstellung des Pfropfungsgrades und die Menge und Art der eingesetzten Monomeren bzw. durch die Verfahrensweise zur Herstellung der Mischpolymerisate ist es möglich, die Eigenschaften der Polyether gezielt zu verändern. Geeignete Monomere sind zum Beispiel Methylmethacrylat, Styrol oder Maleinsäureanhydrid.

Die nach diesen Verfahren erhaltenen Polysiloxane weisen typischerweise Molekulargewichtsverteilungen mit einer Polydispersität von D < 1,6 auf.

Eine Verbreiterung der Molgewichtsverteilung lässt sich am einfachsten herbeiführen, indem Siloxane mit einem unterschiedlichen mittleren Molgewicht miteinander gemischt werden.

Ein Gegenstand der Erfindung ist daher ein Emulgator-System, bei dem die Polydispersität des Polysiloxans durch Mischung von Polysiloxanen mit unterschiedlichen Molekulargewichten eingestellt worden ist.

Daneben ist eine Verbreiterung der Molgewichtsverteilung auch durch eine gezielte Vernetzung der Polyethersiloxane möglich. Zum Beispiel kann dieses erreicht werden, indem mehrfachfunktionelle doppelbindungshaltige Substrate während der Hydrosilylierung der Polyether zugesetzt werden, zum Beispiel Diallylpolyether oder Divinylsiloxane (US 2006/0155090).

Ein weiterer Gegenstand der Erfindung ist daher ein Emulgator-System, bei dem der Dispersionsindex des Polysiloxans dadurch eingestellt worden ist, indem während der Herstellung des Polysiloxans im Rahmen der Hydrosilylierung mehrfachfunktionelle doppelbindungshaltige Substrate eingesetzt wurden.

Eine Vernetzung zur weiteren Verbreiterung der Molekulargewichtsverteilung ist weiterhin möglich, indem die Polyethersiloxane über die Endgruppen der Polyether mit mehrfachfunktionellen reaktiven Substraten, wie Isocyanaten (US 7319120) oder Carbonsäureanhydriden umgesetzt werden.

Daher ist ein weiterer Gegenstand der Erfindung ein Emulgator-System, bei dem die Polydispersität des Polyethersiloxans dadurch eingestellt worden ist, indem die Polyethersiloxane über die Endgruppen der Polyether mit mehrfachfunktionellen reaktiven Substraten umgesetzt wurden.

Eine Kombination der verschiedenen Maßnahmen zur Verbreiterung der Molekulargewichtsverteilung ist natürlich möglich.

Für den Einsatz der erfindungsgemäßen Emulgator-Systeme in kosmetischen Applikationen ist es vorteilhaft, wenn diese bei Raumtemperatur flüssig und pumpbar sind.

Daher ist es vorteilhaft, hochviskose erfindungsgemäße Emulgator-Systeme durch Zusatz von geeigneten Verflüssigungsmitteln in eine pumpbare, flüssige Form zu bringen. Typischerweise haben derartige pumpbare Systeme eine Viskosität von < 10000 mPas (bei einer Scherrate von 10 s⁻¹ bei 25 °C). Vorzugsweise sind diese pumparen verflüssigungsmittelhaltigen Emulgator-Systeme klar bis transluzent-opak.

Als geeignete Verflüssigungsmittel können üblicherweise alle Arten kosmetischer Emollients eingesetzt werden. Als kosmetische Emollients können alle kosmetischen Öle insbesondere Mono- oder Diester von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen eingesetzt werden. Ebenso sind die Veresterungsprodukte aliphatischer, difunktioneller Alkohole mit 2 bis 36 C-Atomen mit monofunktionellen aliphatischen Carbonsäuren mit 1 bis 22 C-Atomen geeignet. Des Weiteren eignen sich langkettige Arylsäureester wie z.B. Ester der Benzoesäure, z.B. Benzoesäureester von linearen oder verzweigten, gesättigten oder ungesättigten Alkoholen mit 1 bis 22 C-Atomen, oder auch Benzoesäureisostearylester oder Benzoesäureoctyldocecylester. Weitere als Emollients und Ölkomponenten geeignete Monoester sind z.B. die Methylester und Isopropylester von Fettsäuren mit 12 bis 22 C-Atomen wie z.B. Methyllaurat, Methylstearat, Methyloleat, Methylerucat, Isopropylpalmitat, Isopropylmyristat, Isopropylstearat, Isopropyloleat. Andere geeignete Monoester sind z.B. n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, z.B. Ester aus ungesättigten Fettalkoholen mit 12 bis 22 C-Atomen und gesättigten und ungesättigten Fettsäuren mit 12 bis 22 C-Atomen, wie sie aus tierischen und pflanzlichen Fetten zugänglich sind. Geeignet sind aber auch natürlich vorkommende Monoester- bzw. Wachsester-Gemische, wie sie z.B. im Jojobaöl oder im Spermöl vorliegen. Geeignete Dicarbonsäureester sind z.B. Di-n-butyl-adipat, Di-n-butylsebacat, Di-(2-ethylhexyl)-adipat, Di-(2-hexyldecyl)-succinat, Di-isotridecylacelaat. Geeignete Diolester sind z.B. Ethylenglycoldioleat, Ethylenglycol-di-isotridecanoat, Propylenglycol-di-(2-ethylhexanoat), Butandiol-diisostearat, Butandiol-di-caprylat/caprat und Neopentylglycol-di-caprylat. Weitere Fettsäureester, die als Emollients eingesetzt werden können, sind z.B. C₁₂-₁₅ Alkylbenzoat, Dicaprylylcarbonat, Diethylhexylcarbonat. Ebenso als Emollients und Ölkomponente können längerkettige Triglyceride, d.h. dreifache Ester des Glycerins mit drei Säuremolekülen, wovon mindestens eine längerkettig ist, eingesetzt werden. Hier seien beispielhaft Fettsäuretriglyceride erwähnt; als solche können beispielsweise natürliche, pflanzliche Öle, z.B. Olivenöl, Sonnenblumenöl, Sojaöl, Erdnußöl, Rapsöl, Mandelöl, Sesamöl, Avocadoöl, Rizinusöl, Kakaobutter, Palmöl aber auch die flüssigen Anteile des Kokosöls oder des Palmkernöls sowie tierische Öle wie z.B. Haifischlebertran, Dorschleberöl, Walöl, Rindertalg und Butterfett, Wachse wie Bienenwachs, Karnaubapalmwachs, Spermazet, Lanolin und Klauenöl, die flüssigen Anteile des Rindertalgs oder auch synthetische Triglyceride von Capryl-Caprinsäure-Gemischen, Triglyceride aus technischer Ölsäure, Triglyceride mit Isostearinsäure, oder aus Palmitinsäure-Ölsäure-Gemischen als Emollients und Ölkomponenten eingesetzt werden. Weiterhin können Kohlenwasserstoffe, insbesondere auch flüssige Paraffine und Isoparaffine eingesetzt werden. Beispiele für einsetzbare Kohlenwasserstoffe sind Paraffinöl, Isohexadecan, Polydecen, Vaseline, Paraffinum perliquidum, Squalan, Ceresin. Weiterhin sind auch lineare oder verzweigte Fettalkohole wie Oleylalkohol oder Octyldodecanol, sowie Fettalkoholether wie Dicaprylyl Ether einsetzbar. Geeignete Siliconöle und -wachse sind z.B. Polydimethylsiloxane, Cyclomethylsiloxane, sowie aryl- oder alkyl- oder alkoxy-substituierte Polymethylsiloxane oder Cyclomethylsiloxane. Als weitere Ölkörper kommen beispielsweise Guerbetalkohole auf Basis von Fettalkoholen mit 6 bis 18, vorzugsweise 8 bis 10 Kohlenstoffatomen, Ester von linearen C6-C22-Fettsäuren mit linearen C6- C22-Fettalkoholen, Ester von verzweigten C6-C13-Carbonsäuren mit linearen C6-C22-Fettalkoholen, Ester von linearen C6-C22-Fettsäuren mit verzweigten C8-C18-Alkoholen, insbesondere 2-Ethylhexanol oder Isononanol, Ester von verzweigten C6-C13-Carbonsäuren mit verzweigten Alkoholen, insbesondere 2-Ethylhexanol oder Isononanol, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C6-C10-Fettsäuren, flüssige Mono-/Di-/Triglyceridmischungen auf Basis von C6-C18-Fettsäuren, Ester von C6-C22-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C6-C22-Fettalkoholcarbonate, Guerbetcarbonate, Ester der Benzoesäure mit linearen und/oder verzweigten C6-C22-Alkoholen (z. B. Finsolv™ TN), Dialkylether, Ringöffnungsprodukte von epoxidierten Fettsäureestern mit Polyolen, Siliconöle und/oder aliphatische bzw. naphthenische Kohlenwasserstoffe in Betracht.

Bevorzugt als Verflüssiger eingesetzte Emollients sind Mono- oder Diester von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen. Ebenso bevorzugt werden längerkettige Triglyceride, d.h. dreifache Ester des Glycerins mit drei Säuremolekülen, wovon mindestens eine längerkettig (Anzahl an C-Atomen größer 1 2) ist, eingesetzt. Ebenso bevorzugt werden verzweigte und unverzweigte flüssige Kohlenwasserstoffe sowie Siliconöle als Verflüssigungsmittel eingesetzt.

Auch Hydrotrope können als Verflüssigungsmittel eingesetzt werden. Hydrotrope sind beispielsweise Ethanol, Isopropylalkohol oder Polyole. Polyole, die hier in Betracht kommen, können 2 bis 15 Kohlenstoffatome und mindestens zwei Hydroxylgruppen besitzen. Typische Beispiele sind:
Glycerin, Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton, technische Oligoglyceringemische mit einem Eigenkondensationsgrad von 1,5 bis 10 wie etwa technische Diglyceringemische mit einem Diglyceringehalt von 40 bis 50 Gew.-%,
Methylolverbindungen, wie insbesondere Trimethylolethan, Trimethylolpropan, Trimethylolbutan, Pentaerythrit und
Dipentaerythrit, Niedrigalkylglucoside, insbesondere solche mit 1 bis 4 Kohlenstoffatomen im Alkylrest, wie beispielsweise Methyl- und Butylglucosid, Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose, Aminozucker, wie beispielsweise Glucamin.

Bevorzugt eingesetzte Hydrotrope als Verflüssigungsmittel sind etwa Glycerin, Propylenglycol, Butylenglycol, Polyethylenglycol oder Polypropylenglycol.

Ein weiterer Gegenstand der Erfindung sind daher flüssige, pumpbare Emulgator-Systeme, die als zusätzliche Komponente ein Verflüssigungsmittel enthalten.

Vorzugsweise sind diese Emulgator-Systeme klar bis transluzent-opak.

Die erfindungsgemäßen Emulgator-Systeme werden als Öl-in-Wasser-Emulgatoren zur Herstellung von kosmetischen und pharmazeutischen Öl-in-Wasser-Emulsionen eingesetzt; sie können daher auch als Dispergierhilfsmittel für Partikel und Pigmente und daher zur Herstellung von Dispersionen verwendet werden.

Als zu dispergierende Partikel und Pigmente kommen beispielsweise feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, z.B. zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Partikel und Pigmente können außerdem micronisierte organische Pigmente, wie beispielsweise 2,2'-Methylene-bis-{6-(2H-benzotriazole-2-yl)-4-(1, 1, 3, 3-tetramethylbutyl)-phenol} mit einer Partikelgröße von < 200 nm sein. Weiterhin können auch Partikel und Pigmente eindispergiert werden, die zu speziellen sensorischen Effekten führen, wie etwa Nylon-12, Bornitrid, Polymerpartikel wie etwa Polyacrylat- oder Polymethylacrylatpartikel oder Siliconelastomere.

Somit sind kosmetische und pharmazeutische Öl-in-Wasser-Emulsionen und Dispersionen enthaltend erfindungsgemäße Emulgator-Systeme ebenfalls Gegenstand der Erfindung.

Die erfindungsgemäßen kosmetischen und pharmazeutischen Emulsionen und Dispersionen enthalten bezogen auf die Gesamtmasse mehr Massenprozent Ölkomponente als die Summe der Massenprozente von Emulgator, Tensid und ggfs. Co-Emulgator.

Bevorzugt werden erfindungsgemäße Emulgator-Systeme zur Herstellung von O/W-Tränkemulsionen für kosmetische Textilien verwendet. Bevorzugt handelt es sich bei den Textilien um Feuchttücher, besonders bevorzugt um kosmetische Feuchttücher.

Somit sind die mit Hilfe der erfindungsgemäßen Emulgator-Systeme erhaltenen O/W-Tränkemulsionen für Textilien ebenfalls Gegenstand der Erfindung.

Ebenso sind die mit erfindungsgemäßen O/W-Tränkemulsionen getränkten Textilien Gegenstand der Erfindung.

Diese zeichnen sich durch eine gute Reinigungsleistung und ein angenehm samtig-glattes Hautgefühl aus.

Ein weiterer Gegenstand der Erfindung ist die Verwendung der erfindungsgemäßen Emulgator-Systeme zur Herstellung kosmetischer, dermatologischer oder pharmazeutischer Formulierungen. Somit ist die kosmetische, dermatologische oder pharmazeutische Formulierung enthaltend mindestens ein erfindungsgemäßes Emulgator-System oder mindestens eine erfindungsgemäße Emulsion oder Dispersion ebenfalls Gegenstand der Erfindung.

Die kosmetischen, dermatologischen oder pharmazeutischen Formulierungen sowie die Pflege- und Reinigungsmittel können z.B. mindestens eine zusätzliche Komponente enthalten, ausgewählt aus der Gruppe der
Emollients,
Emulgatoren und Tenside,
Verdicker/Viskositätsregler/Stabilisatoren,
UV-Lichtschutzfilter,
Antioxidantien,
Hydrotrope (oder Polyole),
Fest- und Füllstoffe,
Filmbildner,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Kosmetische Wirkstoffe,
Pflegeadditive,
Überfettungsmittel,
Lösungsmittel.

Substanzen, die als beispielhafte Vertreter der einzelnen Gruppen eingesetzt werden können, sind der deutschen Anmeldung DE 102008001788.4 zu entnehmen.

In einer bevorzugten Ausführungsform enthalten die erfindungsgemäßen kosmetischen, dermatologischen oder pharmazeutischen Formulierungen als zusätzliche Komponente Partikel oder Pigmente, bevorzugt solche ausgewählt aus der Gruppe Titandioxid, Zinkoxid, Eisenoxid, Aluminiumoxid, Zirkoniumoxid, Silicate (Talk), und Zinkstearat, Nylon-12, Bornitrid, Polyacrylat- oder Polymethylacrylatpartikel oder Siliconelastomere.

In einer ebenso bevorzugten Ausführungsform enthalten die erfindungsgemäßen kosmetischen, dermatologischen oder pharmazeutischen Formulierungen als zusätzliche Komponente kosmetische Wirkstoffe, bevorzugt solche ausgewählt aus der Gruppe: Tocopherol, Tocopherolacetat, Tocopherolpalmitat, Ascorbinsäure, Desoxyribonucleinsäure, Coenzym Q10, Retinol, Bisabolol, Allantoin, Phytantriol, Panthenol, AHA-Säuren, Aminosäuren, Hyaluronsäure, alpha-Hydroxysäuren, Polyglutaminsäure, Creatin (und Creatinderivate), Guanidin (und Guanidinderivate), Ceramide, Phytosphingosin (und Phytosphingosinderivate), Sphingosin (und Sphingosinderivate), Pseudoceramide, Sphingolipide, essentielle Öle, Peptide und Oligopeptide, Proteinhydrolysate, Pflanzenextrakte und Vitaminkomplexe.

Als Applikationsformen der Emulsionen und Dispersionen enthaltend das erfindungsgemäße Emulgator-System sind daher Sprays, Lotionen, Cremes, Salben und somit der Einsatz über einen sehr breiten Konsistenzbereich von wasserdünn bis stark pastös, im Extremfall sogar fest, möglich.

Daher können die Emulgator-Systeme beispielsweise in Pflegecremes und -lotionen für Gesicht, Körper und Hände, in Sonnenschutzemulsionen, in Schminken, in Aersolen, Rollons, Pumpsprays, Stiften z.B. im AP/Deo-Bereich, in Babypflegeprodukten, in Intimpflege-, Fußpflege-, Haarpflege-, Nagelpflege, Zahnpflege- oder Mundpflegeprodukten sowie in dermatologischen Salben eingesetzt werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

Allgemeines Schema 1 zur Herstellung der in den Beispielen eingesetzten Polyethersiloxane:

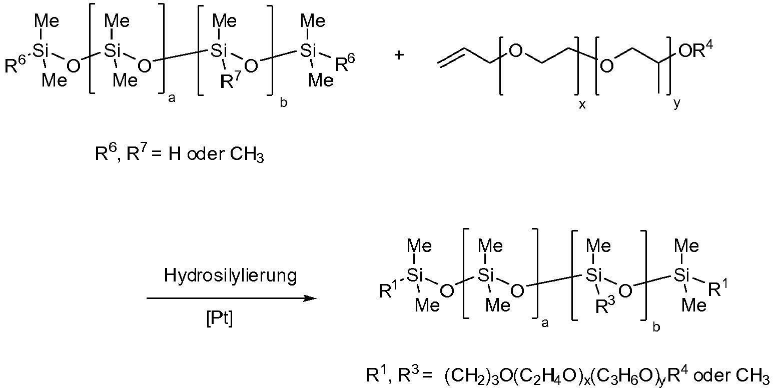

### Emulgator 1:

Nach Schema 1 wurden in einem Vierhalskolben, versehen mit Rührer, Tropftrichter, Thermometer und Rückflusskühler, 48 g (25 mmol SiH) eines SiH-Siloxan (mit R⁶=R⁷=H, a=200, b=5), 17 g (25 mmol SiH) eines zweiten SiH-Siloxans (mit R⁶=CH₃, R⁷=H, a=80, b=10), 101 g (65 mmol) eines Allylpolyethers (mit x=25, y=4, R⁴=CH₃) und 10 ppm Karstedt-Katalysator in 100 ml Toluol bei 95 °C unter Stickstoff umgesetzt. Laut SiH-Wert-Bestimmung wurde nach 2 h vollständiger Umsatz des SiH-Siloxans erhalten. Flüchtige Anteile wurden anschließend im Vakuum bei 120 °C abdestilliert. Es wurde ein viskoses, klares, fast farbloses Produkt erhalten.

### Emulgator 2:

Nach Schema 1 wurden in einem Vierhalskolben, versehen mit Rührer, Tropftrichter, Thermometer und Rückflusskühler, 18 g (10 mmol SiH) eines SiH-Siloxan (mit R⁶=H, R⁷=Me, a=50, b=0), 14 g (20 mmol SiH) eines zweiten SiH-Siloxans (mit R⁶=Me, R⁷=H, a=80, b=10), 63 g (20 mmol SiH) eines dritten SiH-Siloxans (mit R⁶=CH₃, R⁷=H, a=200, b=5), 90 g (65 mmol) eines Allylpolyethers (mit x=20, y=5, R⁴=H) und 10 ppm Karstedt-Katalysator in 50 ml Toluol bei 95 °C unter Stickstoff umgesetzt. Laut SiH-Wert-Bestimmung wurde nach 2 h vollständiger Umsatz des SiH-Siloxans erhalten. Flüchtige Anteile wurden anschließend im Vakuum bei 120 °C abdestilliert. Es wurde ein viskoses, trübes, leicht gelbes Produkt erhalten, welches nach Lagerung phasensepariert. Vor der Verwendung in den Emulsionsversuchen wurde das Produkt durch einfaches Rühren bei Raumtemperatur homogenisiert.

### Emulgator 3:

Produkt "Beispiel 3" wurde durch Vermischen der folgenden, einzeln nach Schema 1 und analog zu Beispiel 1 hergestellten Polyethersiloxane in Capric-/Capryltriglycerid erhalten.

50 g eines ersten Polyethersiloxans (mit R¹=CH₃, R³=PE, x=25, y=4, R⁴=CH₃, a=45, b=5) und 32 g eines zweiten Polyethersiloxans (mit R¹=R³=PE, x=20, y=5, R⁴=H, a=200, b=6) werden in 18 g Capric-/Capryltriglycerid gelöst.

### Emulgator 4:

Produkt "Beispiel 4" wurde durch Vermischen der folgenden, einzeln nach Schema 1 und equivalent zu Beispiel 1 hergestellten Polyethersiloxane in Capric-/Capryltriglycerid erhalten.

50 g eines ersten Polyethersiloxans (mit R¹=CH₃, R³=PE, x=25, y=4, R⁴=CH₃, a=75, b=5) und 32 g eines zweiten Polyethersiloxans (mit R¹=R³=PE, x=20, y=5, R⁴=H, a=200, b=6) werden in 18 g Capric-/Capryltriglycerid gelöst.

### Emulgator 5:

Produkt "Beispiel 5" wurde durch Vermischen der folgenden, einzeln nach Schema 1 und equivalent zu Beispiel 1 hergestellten Polyethersiloxane in Capric-/Capryltriglycerid erhalten.

45 g eines ersten Polyethersiloxans (mit R¹=CH₃, R³=PE, x=25, y=4, R⁴=CH₃, a=45, b=5), 28 g eines zweiten Polyethersiloxans (mit R¹=R³=PE, x=20, y=5, R⁴=H, a=200, b=6) und 11 g eines Polyethersiloxans mit folgender Struktur

[R⁹Me₂SiO_{1/2}]₃[SiMe₂O_{2/2}]₅₀[SiPhO_{3/2}]

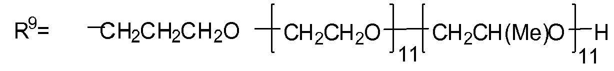

werden in 16 g Capric-/Capryltriglycerid gelöst.

GPC-Daten der Emulgator-Beispiele:

| **Emulgator** | **D** | **Anteil >10⁴ g/mol** | **Anteil >10⁵ g/mol** |
|---|---|---|---|
| 1 | 1,8 | 92,2% | 5,3% |
| 2 | 2,1 | 86,4% | 2,5% |
| 3 | 1, 8 | 86,4% | 2,5% |
| 4 | 1, 8 | 88,8% | 3,9% |
| 5 | 1,9 | 83,8% | 2,7% |

### Vergleichsemulgatoren 1-4 (nicht erfindungsgemäß, zur Abgrenzung vom Stand der Technik):

Die Struktur der Vergleichsemulgatoren 1 bis 3 entspricht der allgemeinen Formel:

R¹(CH₃)₂SiO-[(CH₃)₂SiO]ₐ-[(CH₃)R³SiO]_{b}-Si(CH₃)₂R¹

mit: R¹, R³ = CH₃ oder ein Polyether ("PE") des Typs:
-(CH₂)₃-O-(C₂H₄O)ₓ-(C₃H₆₀)_{y}-R⁴ mit R⁴ = H oder CH₃

| **Vergl. Emulg.** | **a** | **b** | **R¹** | **R³** | **R⁴** | **x** | **y** | **D** | **Anteil >10⁴ g/mol** | **Anteil >10⁵ g/mol** |
|---|---|---|---|---|---|---|---|---|---|---|
| VE1 | 50 | 0 | PE | - | CH₃ | 15 | 10 | 1,3 | 31,0% | < 0,1% |
| VE2 | 100 | 0 | PE | - | H | 11 | 17 | 1,3 | 62,7% | < 0,1% |
| VE3 | 20 | 5 | CH₃ | PE | H | 14 | 4 | 1,5 | 58,1% | < 0,1% |

Die Vergleichsemulgatoren 1 - 2 entsprechen den Beispielen 2 - 3 der EP 1125574.

Bei Vergleichsemulgator V3 handelt es sich um einen typischen kammartig aufgebauten Siliconpolyether. Vergleichsemulgator VE4:
ABIL CARE 85 (INCI: Bis-PEG/PPG-16/16 PEG/PPG-16/16 Dimethicone; Caprylic/Capric Triglyceride; EVONIK Goldschmidt GmbH):
D = 1,4;
Anteil >10⁴ g/mol = 58,4%;
Anteil >10⁵ g/mol = < 0,1%.

### Anwendungsbeispiele:

Alle Konzentrationen in den Anwendungsbeispielen sind in Gewichtsprozent angegeben. Zur Herstellung der Emulsionen wurden dem Fachmann bekannte übliche Homogenisierverfahren eingesetzt.

### Emulgierleistung:

Zur Überprüfung der Emulgierleistung in O/W-Emulsionen wurde ein Schnelltest eingesetzt, der unter sehr kritischen Bedingungen (nur 0,5% Emulgator) sehr schnell zeigt, welche Emulgator-Systeme sich durch hervorragende Emulgieraktivität auszeichnen.

Unter Verwendung üblicher Öle und Stabilisatoren zeigt insbesondere die Stabilität nach 24 h Lagerung bei 50 °C sehr deutlich, ob ein Emulgator-System sehr gute stabilisierende Eigenschaften hat.

Die Ergebnisse der erfindungsgemäßen Emulgator-Systeme 1 bis 5 sind im Vergleich zu den Ergebnissen der Vergleichsemulgatoren 1 bis 4 in Tabelle 1 zusammengefasst. Die Herstellung der Emulsionen erfolgte dabei nach folgendem Verfahren:
Die Phasen A und B werden bei Raumtemperatur gemischt, Phase C wird ohne Rühren zugegeben. Anschließend wird 1 min homogenisiert. Die Phasen D und E werden zugegeben, anschließend wieder 1 min homogenisiert.

Die Ergebnisse der Emulsionsbeispiele 1 bis 5 zeigen, dass die erfindungsgemäßen Emulgatoren deutlich höhere Stabilisierungseigenschaften besitzen als die Vergleichsemulgatoren VE 1 - 4.

**Tabelle 1: Zusammensetzung und Bewertung der Untersuchungen im Emulsionsschnelltest.**

| | **Beispiele** | **1** | **2** | **3** | **4** | **5** |
|---|---|---|---|---|---|---|
| A | Emulgator-system 1 | 0,5% | | | | |
| | Emulgator-system 2 | | 0,5% | | | |
| | Emulgator-system 3 | | | 0,5% | | |
| | Emulgator-system 4 | | | | 0,5% | |
| | Emulgator-system 5 | | | | | 0,5% |
| | Ethylhexyl Stearate | 9,0% | 9,0% | 9,0% | 9,0% | 9,0% |
| | Paraffinum Perliquidum | 9,0% | 9,0% | 9,0% | 9,0% | 9,0% |
| | Ethanol | 5,0% | 5,0% | 5,0% | 5,0% | 5,0% |
| B | Carbomer | 0,16% | 0,16% | 0,16% | 0,16% | 0,16% |
| | Ethylhexyl Stearate | 1,04% | 1,04% | 1,04% | 1,04% | 1,04% |
| C | Demineralized Water | ad 100% | ad 100% | ad 100% | ad 100% | ad 100% |
| D | NaOH (5% solution) | 1,25% | 1,25% | 1,25% | 1,25% | 1,25% |
| E | Euxyl® K 300¹⁾ | 0,70% | 0,70% | 0,70% | 0,70% | 0,70% |
| | | | | | | |
| | Stabilität nach 24 h bei 50°C | stabil | stabil | stabil | stabil | stabil |

| | | | | | | |
|---|---|---|---|---|---|---|
| ¹⁾Euxyl® K 300(Schülke & Mayr): Phenoxyethanol, Methylparaben, Ethylparaben, Propylparaben, Butylparaben, Isopropylparaben | | | | | | |

Vergleichsbeispiele:

| | **Beispiele** | **V1** | **V2** | **V3** | **V4** |
|---|---|---|---|---|---|
| A | Vergleichs-emulgator 1 | 0,5% | | | |
| | Vergleichs-emulgator 2 | | 0,5% | | |
| | Vergleichs-emulgator 3 | | | 0,5% | |
| | Vergleichs-emulgator 4 | | | | 0,5% |
| | Ethylhexyl Stearate | 9,0% | 9,0% | 9,0% | 9,0% |
| | Paraffinum Perliquidum | 9,0% | 9,0% | 9,0% | 9,0% |
| | Ethanol | 5,0% | 5,0% | 5,0% | 5,0% |
| B | Carbomer | 0,16% | 0,16% | 0,16% | 0,16% |
| | Ethylhexyl Stearate | 1,04% | 1,04% | 1,04% | 1,04% |
| C | Demineralized Water | ad 100% | ad 100% | ad 100% | ad 100% |
| D | NaOH (5% solution) | 1,25% | 1,25% | 1,25% | 1,25% |
| E | Euxyl® K 300¹⁾ | 0,70% | 0,70% | 0,70% | 0,70% |
| | | | | | |
| | Stabilität nach 24 h bei 50°C | Ölseparation starke Koaleszenz | Ölseparation starke Koaleszenz | starke Koaleszenz | Ölseparation starke Koaleszenz |

### Hautgefühl und Emulsionsstabilität:

Um Hautgefühl und Stabilität der beiden erfindungsgemäßen Emulgator-Systeme 1 und 2 zu untersuchen, wurden diese in einer Konzentration von 2% in einer kosmetischen Formulierung eingesetzt (Emulsionsbeispiele 6 und 7).

Als Vergleichsbeispiele dienten die Vergleichsemulgatoren 1 bis 4. (Vergleichsemulsionsbeispiele V5 - V8).

Das Hautgefühl der entsprechenden Emulsion wurde in einem Panel von 10 Personen im Vergleich jeweils zur Formulierung mit Vergleichsemulgator 1 bewertet.

Die Testergebnisse sind in Tabelle 2 zusammengefasst.

Die Emulsionsproben wurden zur Beurteilung bei Raumtemperatur, 5°C, 40°C und 45°C gelagert und nach drei Monaten Lagerzeit beurteilt.

Da vor allen Dingen die Lagerung bei 45°C besonders kritisch ist, beschränken sich die Stabilitätsangaben in der Tabelle 2 auf die Beobachtungen bei 45°C.

In diesen Beispielformulierungen wird deutlich, dass es lediglich mit den erfindungsgemäßen Emulgatoren möglich ist, sowohl stabile als auch vom Hautgefühl vorteilhafte Formulierungen herzustellen.

**Tabelle 2: Hautgefühl und Stabilitätsdaten von Testemulsionen**

| **Beispiele** | **6** | **7** | **V5** | **V6** | **V7** | **V8** |
|---|---|---|---|---|---|---|
| Emulgator-system 1 | 2,00% | | | | | |
| Emulgator-system 2 | | 2,00% | | | | |
| Vergleichs-emulgator 1 | | | 2,00% | | | |
| Vergleichs-emulgator 2 | | | | 2,00% | | |
| Vergleichs-emulgator 3 | | | | | 2,00% | |
| Vergleichs-emulgator 4 | | | | | | 2,00% |
| Ethylhexyl Stearate | 10,0% | 10,0% | 10,0% | 10,0% | 10,0% | 10,0% |
| Paraffinum Perliquidum | 9,0% | 9,0% | 9,0% | 9,0% | 9,0% | 9,0% |
| Carbomer | 0,16% | 0,16% | 0,16% | 0,16% | 0,16% | 0,16% |
| Xanthan Gum | 0,16% | 0,16% | 0,16% | 0,16% | 0,16% | 0,16% |
| Demineralized Water | ad 100% | ad 100% | Ad 100% | ad 100% | ad 100% | ad 100% |
| NaOH (5% solution) | 1,25% | 1,25% | 1,25% | 1,25% | 1,25% | 1,25% |
| Euxyl® K 300¹⁾ | 0,70% | 0,70% | 0,70% | 0,70% | 0,70% | 0,70% |
| | | | | | | |
| Stabilität nach 3 Monaten bei 45°C | stabil | stabil | Ölseparation starke Koaleszenz | Ölseparation starke Koaleszenz | starke Koaleszenz | Starke Koaleszenz |
| Hautgefühl | glatt, samtig | weich, seidig, glatt | weich, glatt | weich, glatt, etwas ölig | trocken, klebrig | weich, glatt, samtig |

### Weitere Emulsionsbeispiele:

Diese Beispiele sollen zeigen, dass die erfindungsgemäßen Emulgatoren in einer Vielzahl kosmetischer Formulierungen eingesetzt werden können.

Es ist darüber hinaus mit Hilfe der erfindungsgemäßen Emulgatoren möglich, Pigmente oder Festkörper stabil in Emulsionspräparate einzuarbeiten.

Weiterhin zeigen die Beispiele die gute Kompatibilität mit typischen Coemulgatoren, Ölen, Verdickern und Stabilisatoren.

### O/W Emulsionsbeispiele

**Anti-Aging Tagescreme**

| Beispiel | **8** |
|---|---|
| Emulgatorsystem 1 | 1,50% |
| Ceteareth-25 | 1,00% |
| Stearyl Alcohol | 1,50% |
| Glyceryl Stearate | 3,00% |
| Stearic Acid | 1,50% |
| Myristyl Myristate | 1,00% |
| Ceramide IIIB | 0,10% |
| Caprylic/Capric Triglyceride | 5,00% |
| Ethylhexyl Palmitate | 4,40% |
| Ethylhexyl Methoxycinnamate | 2,00% |
| Butyl Methoxydibenzoylmethane | 1,00% |
| Glycerin | 3,00% |
| Water | ad 100% |
| TEGO® Carbomer 134 (Carbomer) | 0,10% |
| Ethylhexyl Palmitate | 0,40% |
| Sodium Hydroxide (10% in water) | q.s. |
| Preservative | q.s. |
| Parfum | q.s. |

**Selbstbräunende Köperlotion**

| Beispiel | **9** |
|---|---|
| Emulgator-System 3 | 2,00% |
| Cetearyl Isononanoate | 5,00% |
| Decyl Cocoate | 5,00% |
| Isopropyl Myristate | 5,00% |
| Sepigel® 305 (Polyacrylamide; C13-14 Isoparaffin; Laureth-7) | 1,50% |
| PEG-30 Glyceryl Stearate | 2,00% |
| Dihydroxyacetone | 5,00% |
| Propylene Glycol | 3,00% |
| Water | ad 100% |
| Citric Acid | q.s. |
| Preservative | q.s. |
| Parfum | q.s. |

**Kationische Sonnenschutzcreme**

| Beispiel | **10** |
|---|---|
| Emulgator-System 2 | 2,00% |
| Distearyldimonium Chloride | 1,50% |
| Glyceryl Stearate | 2,00% |
| Stearyl Alcohol | 1,00% |
| C12-15 Alkyl Benzoate | 5,00% |
| TEGO® Sun TDEC 45 (Titanium Dioxide; Diethylhexyl Carbonate; Polyglyceryl-6 Polyhydroxystearate)) | 5,00% |
| Diethylhexyl Carbonate | 3,50% |
| Cetyl Ricinoleate | 1,00% |
| Triisostearin | 1,00% |
| Octocrylene | 3,00% |
| Ethylhexyl Methoxycinnamate | 4,00% |
| Butyl Methoxydibenzoylmethane | 2,00% |
| Water | ad 100% |
| Glycerin | 3,00% |
| Preservative | q.s. |
| Parfum | q.s. |

**Hautglättende Bodylotion**

| Beispiel | **11** |
|---|---|
| Emulgatorsytem 5 | 2,50% |
| Diethylhexyl Carbonate | 7,00% |
| Isopropyl Palmitate | 7,60% |
| Creatine | 0,50% |
| Panthenol | 0,50% |
| Glycerin | 3,00% |
| Water | ad 100% |
| TEGO® Carbomer 341 ER (Acrylates / C10-30 Alkyl Acrylate Cross-polymer) | 0,30% |
| Xanthan Gum | 0,10% |
| Sodium Hydroxide (10% in water) | q.s. |
| TEGO® Smooth Complex (Betaine; Urea; Potassium Lactate; Polyglutamic Acid; Hydrolyzed Sclerotium Gum) | 2,00% |
| Preservative | q.s. |
| Parfum | q.s. |

**Seidiges Cremegel**

| Beispiel | **12** |
|---|---|
| Emulgator-System 3 | 2,00% |
| Bis-PEG/PPG-14/14 Dimethicone | 2,00% |
| Cyclomethicone | 10,00% |
| Dimethicone | 3,00% |
| Cetyl Ricinoleate | 2,00% |
| Xanthan Gum | 0,20% |
| TEGO® Carbomer 341 ER (Acrylates / C10-30 Alkyl Acrylate Cross-polymer) | 0,40% |
| Caprylic/Capric Triglyceride | 1,90% |
| Water | ad 100% |
| PEG/PPG-20/20 Dimethicone | 1,00% |
| Alcohol | 5,00% |
| Sodium Hydroxide (10% in water) | q.s. |
| Preservative | q.s. |
| Parfum | q.s. |

**O/W Tränkemulsion für kosmetische Feuchttücher**

| | Beispiel | **13** |
|---|---|---|
| A | TEGO® Wipe DE (Diethylhexyl Carbonate; Polyglyceryl-4 Laurate; Phenoxyethanol; Methyl-paraben; Dilauryl Citrate; Ethylparaben; Butylparaben; Propylparaben; Isobutylparaben) | 5,70% |
| B | Demineralized water | 5,70% |
| C | Emulgator-System 1 | 0,30% |
| | Creatine | 0,25% |
| | Panthenol | 0,50% |
| | Demineralized water | 93,25 |
| Z | Parfum | q.s. |

Herstellung: Bei Raumtemperatur wird zunächst A mit B gemischt, dann werden unter Rühren C und Z zugegeben.

## Patentansprüche

1. Emulgator-System für kosmetische und pharmazeutische Öl-in-Wasser-Emulsionen enthaltend hochmolekulare organomodifizierte Polysiloxane mit einer Polydispersität größer 1,6, **dadurch gekennzeichnet, dass** der Anteil an Polysiloxanen mit einem Molekulargewicht von ≥ 1*10⁴ g/mol bei über 75% bezogen auf die Gesamtmenge des Polysiloxans liegt und der Anteil an Polysiloxanen mit einem Molekulargewicht von ≥ 1*10⁵ g/mol bei über 2% bezogen auf die Gesamtmenge des Polysiloxans liegt.

2. Emulgator-System gemäß Anspruch 1, **dadurch gekennzeichnet, dass** das Polysiloxan ein Polyethersiloxan ist.

3. Emulgator-System gemäß Anspruch 1 oder 2, **dadurch gekennzeichnet, dass** das Polysiloxan ein Polyethersiloxan der allgemeine Formel I ist
M_{2+c+2d} Dₐ D'_{b} T_{c} Q_{d} Formel I,
wobei
M = (R¹R²₂ SiO_{1/2})
D = (R²₂ Si O_{2/2})
D' = (R²R³ Si O_{2/2})
T = (R² Si O_{3/2})
Q = (Si O_{4/2})
a = 30 - 800, bevorzugt 40 bis 500, insbesondere 50 bis 400,
b = 1 bis 15, bevorzugt 3 bis 10, insbesondere 4 bis 8,
c = 0 bis 2, bevorzugt 0 bis 1, insbesondere 0,
d = 0 bis 2, bevorzugt 0 bis 1, insbesondere 0,
R¹ = R² oder R³,
R² = unabhängig voneinander gleiche oder verschiedene lineare oder verzweigte, gegebenenfalls aromatische Kohlenwasserstoffreste mit 1 bis 16 Kohlenstoffatomen, die gegebenenfalls OH- oder Esterfunktionen tragen bevorzugt Methyl oder Phenyl, insbesondere Methyl,
R³ = unabhängig voneinander gleiche oder verschiedene Polyetherreste der allgemeinen Formel II
-CH₂-CH₂-(CH₂)ₙO(EO)ₓ(PO)_{y}(XO)_{z} R⁴ Formel II,
mit
EO = (C₂H₄O)
PO = (C₃H₆O)
XO = (C₂H₃R⁵O)
n = 1 - 9, insbesondere 1
x = 2 - 50, insbesondere 10-30
y = 0 - 50, insbesondere 2-15
z = 0 - 10, insbesondere 0
R⁴ = unabhängig voneinander gleiche oder verschiedene Reste ausgewählt aus der Gruppe umfassend: H, Alkylreste mit 1 bis 16 C-Atomen, oder Carboxylatreste und
R⁵ = unabhängig voneinander gleiche oder verschiedene Reste ausgewählt aus der Gruppe umfassend: Alkylreste mit 2 bis 16 C-Atomen, die gegebenenfalls durch Etherfunktionen unterbrochen sind, Alkarylreste mit 7 - 18 C-Atomen, Arylreste mit 6 bis 16 C-Atomen, bevorzugt Ethyl oder Phenyl
ist.

4. Emulgator-System gemäß Anspruch 3, **dadurch gekennzeichnet, dass** x/(y+z) > 1 ist.

5. Emulgator-System gemäß mindestens einem der Ansprüche 3 oder 4, **dadurch gekennzeichnet, dass** das Verhältnis a/b > 5 ist.

6. Emulgator-System gemäß mindestens einem der Ansprüche 3 bis 5, **dadurch gekennzeichnet, dass** das Polyethersiloxan der allgemeinen Formel I im Schnitt mindestes drei Polyetherreste enthält und die maximale Anzahl der ans Molekül gebundenen Polyetherreste R³ < a/5 ist.

7. Emulgator-System gemäß mindestens einem der Ansprüche 1 bis 6, **dadurch gekennzeichnet, dass** die Polydispersität des Polysiloxans durch Mischung von Polysiloxanen mit unterschiedlichen Molekulargewichten eingestellt worden ist.

8. Emulgator-System gemäß mindestens einem der Ansprüche 1 bis 7, **dadurch gekennzeichnet, dass** die Polydispersität des Polyethersiloxans dadurch eingestellt worden ist, indem die Polyethersiloxane über die Endgruppen der Polyether mit mehrfachfunktionellen reaktiven Substraten umgesetzt wurden.

9. Emulgator-System gemäß mindestens einem der Ansprüche 1 bis 7, **dadurch gekennzeichnet, dass** die Polydispersität des Polysiloxans dadurch eingestellt worden ist, indem während der Herstellung des Polysiloxans im Rahmen der Hydrosilylierung mehrfachfunktionelle doppelbindungshaltige Substrate eingesetzt wurden.

10. Flüssiges, pumpbares Emulgator-System gemäß mindestens eines der Ansprüche 1 bis 9, **dadurch gekennzeichnet, dass** es als zusätzliche Komponente ein Verflüssigungsmittel enthält.

11. Verwendung mindestens eines der Emulgator-Systeme gemäß mindestens einem der Ansprüche 1 bis 10 zur Herstellung kosmetischer oder pharmazeutischer Öl-in-Wasser-Emulsionen oder Dispersionen.

12. Kosmetische oder pharmazeutische Öl-in-Wasser-Emulsion oder Dispersion enthaltend mindestens eines der Emulgator-Systeme gemäß mindestens einem der Ansprüche 1 bis 10.

13. O/W-Tränkemulsionen für kosmetische Textilien erhältlich durch die Verwendung gemäß Anspruch 11.

14. Verwendung mindestens eines der Emulgator-Systeme gemäß mindestens einem der Ansprüche 1 bis 10 oder mindestens einer kosmetischen oder pharmazeutischen Öl-in-Wasser-Emulsion oder Dispersion gemäß Anspruch 12 oder einer Tränkemulsion gemäß Anspruch 13 zur Herstellung kosmetischer, dermatologischer oder pharmazeutischer Formulierungen.

15. Kosmetische, dermatologische oder pharmazeutische Formulierung erhältlich durch die Verwendung gemäß Anspruch 14.

16. Kosmetische, dermatologische oder pharmazeutische Formulierung gemäß Anspruch 15 als zusätzliche Komponente enthaltend Partikel oder Pigmente.

17. Kosmetische, dermatologische oder pharmazeutische Formulierung gemäß Anspruch 15 als zusätzliche Komponente enthaltend kosmetische Wirkstoffe.

## Claims

1. Emulsifier system for cosmetic and pharmaceutical oil-in-water emulsions comprising high molecular weight organomodified polysiloxanes with a polydispersity greater than 1.6, **characterized in that** the fraction of polysiloxanes with a molecular weight of ≥ 1*10⁴ g/mol is above 75%, based on the total amount of the polysiloxane and the fraction of polysiloxanes with a molecular weight of ≥ 1*10⁵ g/mol is above 2%, based on the total amount of the polysiloxane.

2. Emulsifier system according to Claim 1, **characterized in that** the polysiloxane is a polyethersiloxane.

3. Emulsifier system according to Claim 1 or 2, **characterized in that** the polysiloxane is a polyethersiloxane of the general formula I
M_{2+c+2d} Dₐ D'_{b} T_{c} Q_{d} formula I,
where
M = (R¹R²₂ SiO_{1/2})
D = (R²₂ Si O_{2/2})
D' = (R²R³ Si O_{2/2})
T = (R² Si O_{3/2})
Q = (Si O_{4/2})
a = 30 - 800, preferably 40 to 500, in particular 50 to 400,
b = 1 to 15, preferably 3 to 10, in particular 4 to 8,
c = 0 to 2, preferably 0 to 1, in particular 0,
d = 0 to 2, preferably 0 to 1, in particular 0,
R¹ = R² or R³,
R² = independently of one another, identical or different linear or branched, optionally aromatic hydrocarbon radicals having 1 to 16 carbon atoms, which optionally carry OH or ester functions, preferably methyl or phenyl, in particular methyl,
R³ = independently of one another, identical or different polyether radicals of the general formula II
-CH₂-CH₂-(CH₂)ₙO(EO)ₓ(PO)_{y}(XO)_{z} R⁴ formula II,
where
EO = (C₂H₄O)
PO = (C₃H₆O)
XO = (C₂H₃R⁵O)
n = 1 - 9, in particular 1
x = 2 - 50, in particular 10-30
y = 0 - 50, in particular 2-15
z = 0 - 10, in particular 0
R⁴ = independently of one another, identical or different radicals selected from the group comprising: H, alkyl radicals having 1 to 16 carbon atoms, or carboxylate radicals and
R⁵ = independently of one another, identical or different radicals selected from the group comprising: alkyl radicals having 2 to 16 carbon atoms, which are optionally interrupted by ether functions, alkaryl radicals having 7 - 18 carbon atoms, aryl radicals having 6 to 16 carbon atoms, preferably ethyl or phenyl.

4. Emulsifier system according to Claim 3, **characterized in that** x/(y+z) is > 1.

5. Emulsifier system according to at least one of Claims 3 or 4, **characterized in that** the ratio a/b is > 5.

6. Emulsifier system according to at least one of Claims 3 to 5, **characterized in that** the polyethersiloxane of the general formula I comprises, on average, at least three polyether radicals, and the maximum number of polyether radicals R³ bonded to the molecule is < a/5.

7. Emulsifier system according to at least one of Claims 1 to 6, **characterized in that** the polydispersity of the polysiloxane has been adjusted by mixing polysiloxanes with differing molecular weights.

8. Emulsifier system according to at least one of Claims 1 to 7, **characterized in that** the polydispersity of the polyethersiloxane has been adjusted by reacting the polyethersiloxanes with polyfunctional reactive substrates via the end groups of the polyethers.

9. Emulsifier system according to at least one of Claims 1 to 7, **characterized in that** the polydispersity of the polysiloxane has been adjusted by using polyfunctional double-bond-containing substrates during the preparation of the polysiloxane in the course of the hydrosilylation.

10. Liquid, pumpable emulsifier system according to at least one of Claims 1 to 9, **characterized in that** it comprises a liquefying agent as additional component.

11. Use of at least one of the emulsifier systems according to at least one of Claims 1 to 10 for producing cosmetic or pharmaceutical oil-in-water emulsions or dispersions.

12. Cosmetic or pharmaceutical oil-in-water emulsion or dispersion comprising at least one of the emulsifier systems according to at least one of Claims 1 to 10.

13. O/W impregnation emulsions for cosmetic textiles obtainable through the use according to Claim 11.

14. Use of at least one of the emulsifier systems according to at least one of Claims 1 to 10 or at least one cosmetic or pharmaceutical oil-in-water emulsion or dispersion according to Claim 12 or an impregnation emulsion according to Claim 13 for producing cosmetic, dermatological or pharmaceutical formulations.

15. Cosmetic, dermatological or pharmaceutical formulation obtainable through the use according to Claim 14.

16. Cosmetic, dermatological or pharmaceutical formulation according to Claim 15 as additional component comprising particles or pigments.

17. Cosmetic, dermatological or pharmaceutical formulation according to Claim 15 as additional component comprising cosmetic active ingredients.

## Revendications

1. Système émulsifiant pour des émulsions huile dans eau cosmétiques et pharmaceutiques comprenant des polysiloxanes organomodifiés de poids moléculaire élevé ayant une polydispersité supérieure à 1,6, **caractérisé en ce que** la fraction de polysiloxanes ayant un poids moléculaire ≥ 1*10⁴ g/mol est supérieure à 75 %, sur la base de la quantité totale du polysiloxane et la fraction de polysiloxanes ayant un poids moléculaire ≥ 1*10⁵ g/mol est supérieure à 2 %, sur la base de la quantité totale du polysiloxane.

2. Système émulsifiant selon la revendication 1, **caractérisé en ce que** le polysiloxane est un polyéthersiloxane.

3. Système émulsifiant selon la revendication 1 ou 2, **caractérisé en ce que** le polysiloxane est un polyéthersiloxane de formule générale I
M_{2+c+2d} Dₐ D'_{b} Tₑ Q_{d} formule I,
où
M = (R¹R²₂ SiO_{1/2})
D = (R²₂ Si O_{2/2})
D' = (R²R³ Si O_{2/2})
T = (R² Si O_{3/2})
Q = (Si O_{4/2})
a = 30 à 800, de préférence 40 à 500, en particulier 50 à 400,
b = 1 à 15, de préférence 3 à 10, en particulier 4 à 8,
c = 0 à 2, de préférence 0 à 1, en particulier 0,
d = 0 à 2, de préférence 0 à 1, en particulier 0,
R¹ = R² ou R³,
R² = indépendamment les uns des autres, des radicaux hydrocarbonés facultativement aromatiques, linéaires ou ramifiés, identiques ou différents ayant 1 à 16 atomes de carbone, qui comportent facultativement des fonctions OH ou ester, de préférence méthyle ou phényle, en particulier méthyle,
R³ = indépendamment les uns des autres, des radicaux polyéther identiques ou différents de formule générale II
-CH₂-CH₂-(CH₂)ₙO(EO)ₓ(PO)_{y}(XO)_{z} R⁴ formule II,
où
EO = (C₂H₄O)
PO = (C₃H₆O)
XO = (C₂H₃R⁵O)
n = 1 à 9, en particulier 1
x = 2 à 50, en particulier 10 à 30
y = 0 à 50, en particulier 2 à 15
z = 0 à 10, en particulier 0
R⁴ = indépendamment les uns des autres, des radicaux identiques ou différents choisis dans le groupe comprenant : H, des radicaux alkyle ayant 1 à 16 atomes de carbone, ou des radicaux carboxylate et
R⁵ = indépendamment les uns des autres, des radicaux identiques ou différents choisis dans le groupe comprenant : des radicaux alkyle ayant 2 à 16 atomes de carbone, qui sont facultativement interrompus par des fonctions éther, des radicaux alkaryle ayant 7 à 18 atomes de carbone, des radicaux aryle ayant 6 à 16 atomes de carbone, de préférence éthyle ou phényle.

4. Système émulsifiant selon la revendication 3, **caractérisé en ce que** x/(y+z) est > 1.

5. Système émulsifiant selon au moins une des revendications 3 ou 4, **caractérisé en ce que** le rapport a/b est > 5.

6. Système émulsifiant selon au moins une des revendications 3 à 5, **caractérisé en ce que** le polyéthersiloxane de formule générale I comprend, en moyenne, au moins trois radicaux polyéther, et le nombre maximal de radicaux polyéther R³ liés à la molécule est < a/5.

7. Système émulsifiant selon au moins une des revendications 1 à 6, **caractérisé en ce que** la polydispersité du polysiloxane a été ajustée par mélange de polysiloxanes ayant différents poids moléculaires.

8. Système émulsifiant selon au moins une des revendications 1 à 7, **caractérisé en ce que** la polydispersité du polyéthersiloxane a été ajustée par réaction des polyéthersiloxanes avec des substrats réactifs polyfonctionnels par l'intermédiaire des groupes terminaux des polyéthers.

9. Système émulsifiant selon au moins une des revendications 1 à 7, **caractérisé en ce que** la polydispersité du polysiloxane a été ajustée par utilisation de substrats polyfonctionnels contenant une double liaison pendant la préparation du polysiloxane au cours de l'hydrosilylation.

10. Système émulsifiant liquide, pompable selon au moins une des revendications 1 à 9, **caractérisé en ce qu'**il comprend un agent de liquéfaction en tant que composant additionnel.

11. Utilisation d'au moins un des systèmes émulsifiants selon au moins une des revendications 1 à 10 pour produire des émulsions ou dispersions huile dans eau cosmétiques ou pharmaceutiques.

12. Émulsion ou dispersion huile dans eau cosmétique ou pharmaceutique comprenant au moins l'un des systèmes émulsifiants selon au moins une des revendications 1 à 10.

13. Émulsions d'imprégnation H/E pour textiles cosmétiques pouvant être obtenues par l'utilisation selon la revendication 11.

14. Utilisation d'au moins l'un des systèmes émulsifiants selon au moins une des revendications 1 à 10 ou d'au moins une émulsion ou dispersion huile dans eau cosmétique ou pharmaceutique selon la revendication 12 ou d'une émulsion d'imprégnation selon la revendication 13 pour produire des formulations cosmétiques, dermatologiques ou pharmaceutiques.

15. Formulation cosmétique, dermatologique ou pharmaceutique pouvant être obtenue par l'utilisation selon la revendication 14.

16. Formulation cosmétique, dermatologique ou pharmaceutique selon la revendication 15 en tant que composant additionnel comprenant des particules ou des pigments.

17. Formulation cosmétique, dermatologique ou pharmaceutique selon la revendication 15 en tant que composant supplémentaire comprenant des substances actives cosmétiques.
